# FASCICULE DE BREVET EUROPEEN

(11) **EP 0 987 010 B1**
(45) Date de publication et mention de la délivrance du brevet: **08.09.2004**
(21) Numéro de dépôt: 99401716.8
(22) Date de dépôt: 08.07.1999
(51) Int. Cl.: A61K 7/48, A61K 7/06

(54) **Composition cosmétique contenant au moins un polysaccharide provenant de bactéries d'origine hydrothermale**
Kosmetische zusammensetzung enthaltend einen polysacchariden von einem hydrothermalen bakterium
Cosmetic composition containing at least a polysaccharide from an hydrothermal bacterium

(30) Priorité: 31.07.1998 FR 9810034
(43) Date de publication de la demande: 22.03.2000
(73) Titulaire: Lanatech Laboratoire Nature et Technique, 75009 Paris (FR)
(72) Inventeur: Fristsch, Marie-Claire, c/o I.R.I.S., 75009 Paris (FR); Vacher, Anne-Marie, 78150 Le Chesnay (FR)
(74) Mandataire: de Saint-Palais, Arnaud Marie

(56) Documents cités:
- FR-A- 2 701 488
- STN, Serveur de Bases de Données, Karlsruhe, DE, Fichier Chemical Abstracts, Vol 127, AN=2951 XP002125171
- STN, Serveur de Bases de Données, Karlsruhe, DE, Fichier Chemical Abstracts, Vol 124, AN=81922 XP002125172

## Description

La présente invention concerne une composition cosmétique incluant au moins un polysaccharide provenant de bactéries d'origine hydrothermale.

Elle résulte de l'étude conduite par la demanderesse d'une collection tout à fait particulière de bactéries, à savoir les bactéries mésophiles d'origine hydrothermale.

D'une manière générale, on sait que dans les années 70, les biologistes océanographes participant à des campagnes océanographiques en mer profonde ont découvert, à leur plus grande surprise, l'existence de communautés écologiques originales, vivant au voisinage des bouches hydrothermales. Ces sources hydrothermales propres aux dorsales océaniques actives, ont pour origine les infiltrations d'eau de mer à travers le réseau de failles qui la font circuler dans l'épaisseur de la croûte terrestre, à proximité du magma. Alors que les différentes missions passées avaient montré qu'au delà de 2500 mètres de profondeur la faune n'était guère abondante, les scientifiques ont eu la surprise de découvrir au niveau de ces sources hydrothermales, une faune exubérante de mollusques, de vers, de crustacés, ainsi que des associations complexes de bactéries et d'invertébrés.

Une importante collection de bactéries s'est peu à peu constituée, au fur et à mesure de ces expéditions. Ces espèces ont été décrites, et si la plupart, non pathogènes, appartiennent à des genres connus, il s'agit bien toutefois d'espèces nouvelles. Certaines de ces bactéries, vivant et se reproduisant à de très grandes profondeurs et dans des conditions extrêmes, se sont avérées capables de croître en conditions de culture de laboratoire, de synthétiser et d'excréter dans le milieu de culture un certain nombre de molécules dont l'étude est du plus grand intérêt.

Par ailleurs, il est notoirement connu que certains polysaccharides extraits de champignons, d'algues, de parois de levures ou de bactéries terrestres, sont connus en thérapeutique médicale pour leur action stimulante sur les macrophages dont ils améliorent l'action bactéricide et tumoricide. Ainsi, ces molécules sont capables de stimuler le système immunitaire, permettant de prévenir un certain nombre d'affections.

Certains de ces polysaccharides extraits de parois cellulaires de différents organismes ou micro-organismes, montrent donc des activités biologiques intéressantes. Ces propriétés biologiques ont pu être en partie exploitées dans le domaine cosmétique. Il a été montré par exemple qu'un β-glucan extrait de paroi d'une levure *Saccharomyces cerevisiae* permettait une régénération de la peau.

Les études conduites par la demanderesse avaient plus précisément pour but de déterminer l'activité, dans le domaine cosmétique, d'exopolysaccharides issus de la fermentation de bactéries mesophiles d'origine hydrothermale.

Ces polysaccharides synthétisés par ces bactéries cultivées en laboratoire, sont des polymères de haut poids moléculaire (de 100 000 daltons à plus d'un million de daltons). Ils sont constitués d'enchaînement de sucres variés, neutres ou acides, l'unité monomérique de base représentant généralement 4 à 10 résidus. Certains sont linéaires, mais la plupart sont ramifiés. D'autres sont de structure tout à fait inconnue à l'heure actuelle.

Pour les besoins de l'étude, la demanderesse a effectué des études d'efficacité sur trois polysaccharides très différents les uns des autres d'un point de vue chimique et issus de la fermentation de trois espèces distinctes de bactéries mésophiles d'origine hydrothermale, à savoir :
- deux exopolysaccharides POL.1, POL.2 consistent en des polymères natifs (c'est-à-dire non modifiés) avec un ratio sucres neutres/sucres acides différent, ces deux exopolysaccharides présentant un poids moléculaire très élevé (500 000 à 1 million),
- un polysaccharide modifié POL.3, sulfaté chimiquement, puis dépolymérisé, qui présente un poids moléculaire beaucoup plus faible.

Concernant ces études, il convient préalablement de rappeler que la peau constitue la toute première barrière de l'organisme contre les agressions extérieures. Les kératinocytes, qui représentent la majorité des cellules de l'épiderme, sont engagés dans un programme de différenciation et de maturation extrêmement précis, soumis à des interactions multiples entre les compartiments épidermiques et dermiques. Ce processus aboutit à l'élaboration des kératines et des lipides complexes qui assurent le rôle et l'intégrité de l'épiderme et de sa composante "barrière" : la couche cornée. Il s'agit ici du rôle classique et bien connu de protection mécanique des kératinocytes.

Bien plus intéressante est l'implication de ces mêmes cellules, dans le processus de défense immunitaire cutané. Il est aujourd'hui démontré que les kératinocytes sont la source principale, dans l'épiderme de médiateurs de communication cellulaire. Ils sont aujourd'hui reconnus, au même titre que les cellules de Langerhans, comme des acteurs essentiels et fondamentaux de ce système de défense. Les différentes cellules de la peau agissent en parfaite harmonie grâce à un système de communication intercellulaire très élaboré constitué entre autre par le réseau cytokinique. Les cytokines jouent un rôle majeur dans le maintien d'un état immunitaire normal et sont également impliquées dans l'inflammation, la cicatrisation, l'angiogénèse, l'allergie ou l'apoptose. L'expression régulée de chacune d'elles permet de maintenir un état d'équilibre au niveau de la prolifération et du métabolisme cellulaire local.

Les agressions chimiques ou physiques (radiations ultraviolettes, agents infectieux,...) sont capables de perturber cet équilibre. Il en résulte à la longue une peau abîmée, desséchée, irritée, ... prématurément vieillie, qui de plus possède de moins en moins la capacité à assurer sa propre défense et son rôle de barrière protectrice.

D'où l'intérêt en cosmétique, de chercher à améliorer le système de défense et de protection des cellules de la peau. Cette modulation du système de défense immunitaire cutané peut être obtenue par application topique de molécules capables de stimuler les kératinocytes, induisant ainsi de façon spécifique l'expression de certaines cytokines. Ceci aboutit à une mise en éveil du système de défense immunitaire de la peau, qui est alors capable de réagir mieux et plus vite aux agressions extérieures.

Les recherches effectuées par la demanderesse visaient à mettre en évidence un éventuel effet stimulant de ces polysaccharides sur l'expression de l'Interleukine-1α, par les kératinocytes. L'Interleukine-1α est le tout premier médiateur de communication cellulaire synthétisé et excrété par les kératinocytes, médiateur capable d'initier la "cascade immunitaire".

Ces propriétés d'activation ont été évaluées sur des kératinocytes humains en cultures primaires, selon les trois protocoles expérimentaux différents suivants :

### Protocole 1 (effet préventif sur keratinocytes):

Sur kératinocytes humains en culture. Préincubation 3 heures avec les produits à l'essai, puis stimulation au phorbol myristate acétate (PMA). Dosage de l'IL-1α par un test immunoenzymatique (valeurs en pg/ml) aux temps T1h, T6h, T24h après stimulation. Les résultats figurent sur le tableau I ci-après.

**Tableau I**

| Concentrations | 0 | 0,1 µg/ml | 0,5 µg/ml | 1 µg/ml | 5 µg/ml | 10 µg/ml |
|---|---|---|---|---|---|---|
| POL.1 - T1h | 48 | 50 | 46 | 26 | 17 | 36 |
| POL.1 - T6h | 34 | **68** | 48 | **86** | **191** | **72** |
| POL.1 - T24h | 55 | 31 | 65 | 38 | **79** | 36 |
| POL.2 - T1h | 48 | 47 | 33 | 40 | 26 | 21 |
| POL.2 - T6h | 34 | 44 | 46 | **149** | **88** | **54** |
| POL.2 - T24h | 55 | 25 | 24 | 25 | 60 | 49 |
| POL.3 - T1h | 3 | **16** | 8 | **115** | **37** | **33** |
| POL.3 - T6h | 40 | 43 | **69** | **77** | **78** | **75** |
| POL.3 - T24h | 54 | 60 | 24 | 64 | **93** | **75** |

### Protocole 2 : Sur kératinocytes humains en culture. Préincubation 24 heures avec les produits à l'essai, puis costimulation Produits à l'essai/PMA. Dosage de l'IL-1α (valeurs en pg/ml) au temps T48h après stimulation. Les résultats figurent sur le tableau II ci-après :

**Tableau II**

| Concentrations | 0 | 0,5 µg/ml | 1 µg/ml | 5 µg/ml | 10 µg/ml | 50 µg/ml |
|---|---|---|---|---|---|---|
| POL.1 - T48h | 41 | **86** | **64** | 28 | 29 | 28 |
| POL.2 - T48h | 41 | 33 | 35 | 24 | 21 | 31 |
| POL.3 - T48h | 41 | 31 | 27 | 29 | 34 | 25 |

### Protocole 3 (effet curatif sur kératinocytes) :

Sur kératinocytes humains en culture. Costimulation Produits à l'essai/PMA. Dosage de l'IL-1α (valeurs en pg/ml) aux temps T1h, T3h, T6h après stimulation. Les résultats figurent sur le tableau III ci-après :

**Tableau III**

| Concentrations | 0 | 0,1 µg/ml | 0,5 µg/ml | 1 µg/ml | 5 µg/ml |
|---|---|---|---|---|---|
| POL.1 - T1h | 62 | **86** | **93** | 52 | 47 |
| POL.1 - T3h | 67 | 43 | 65 | 47 | 66 |
| POL.1 - T6h | 30 | 29 | 31 | **45** | 34 |
| POL.2 - T1h | 62 | 64 | **106** | 56 | 46 |
| POL.2 - T3h | 67 | 53 | 54 | 48 | 46 |
| POL.2 - T6h | 30 | 33 | **40** | **60** | 39 |
| POL.3 - T1h | 62 | 46 | 51 | 52 | 43 |
| POL.3 - T3h | 67 | 54 | 56 | 53 | 44 |
| POL.3 - T6h | 30 | 38 | **48** | **61** | **46** |

Les données acquises lors de la mise en oeuvre de ces différents protocoles d'études, permettent de conclure à une action stimulante des 3 polysaccharides testés, sur l'expression de l'Interleukine-1α par les kératinocytes humains en culture. De plus ces produits ne présentent pas de toxicité cellulaire aux doses testées.

Ces trois polysaccharides, de nature chimique fort différente, présentent donc dans ce modèle expérimental in-vitro, une activité biologique incontestable. Forts de cette constatation, la demanderesse a alors cherché à montrer les conséquences positives pour la peau d'une telle activité biologique. Différentes études ont donc été mises en oeuvre, in-vitro sur kératinocytes en culture, ex-vivo sur explants de peau humaine, ou encore in-vivo sur modèle animal. Ceci dans le but de mettre en évidence un éventuel effet protecteur des polysaccharides en test, face à diverses agressions sur la peau.

Des études de cytoxicité des polysaccharides POL.1, POL.2, POL.3 ont été faites vis-à-vis d'un micro-organisme, typiquement du Candida albicans au niveau de keratinocytes humains en culture.

On rappelle à ce sujet que les kératinocytes dans certaines circonstances, possèdent une capacité de phagocytose, qui leur permet de participer très activement à la lutte de l'organisme contre les agressions microbiennes. Il a été démontré in vitro, sur kératinocytes humains, que ceux-ci sont capables de détruire un micro-organisme: *Candida albicans.* Le mécanisme d'action impliqué, fait très certainement intervenir des molécules sécrétées par le kératinocyte à l'encontre de l'élément étranger. Il n'y a donc pas réelle phagocytose du micro-organisme, mais cytotoxicité de kératinocyte vis à vis de celui-ci. Le kératinocyte est alors capable d'ingérer et d'éliminer les débris de cellules tuées.

La peau est soumises de façon quotidienne aux agressions microbiennes de toute origine : levures, moisissures, champignons microscopiques, bactéries ... Certaines germes sont inoffensifs pour la peau, d'autres, bénéfiques pour celle-ci participent à l'équilibre de la flore saprophyte cutanée, d'autres enfin sont pathogènes. Le développement de germes pathogènes à la surface cutanée conduit bien évidemment à un déséquilibre de la flore cutanée et à l'apparition de pathologies de la peau plus ou moins graves, qui nécessitent un traitement médical.

D'où l'intérêt en cosmétique de chercher à prévenir ce type de problèmes, en tentant d'aider la peau à se défendre mieux et plus vite, contre tout début d'agression microbienne. La demanderesse a donc cherché à mettre en évidence l'action stimulante des polysaccharides sur la capacité de destruction de différents micro-organismes néfastes pour la peau, par les kératinocytes.

La mesure de la toxicité contre *Candida* a été réalisée selon une modification de la méthode de Lehrer & Cline (Interaction of *Candida albicans* with human leukocytes & serum, J. Bacteriol. 1969:98:996). Une suspension de *Candida albicans* (0656CBS Delf 4 × 10⁷ cell. dans du PBS) a été incubée en présence de la suspension de kératinocytes et en présence ou non des différentes doses des polysaccharides à l'essai. L'incubation a été poursuivie pendant une heure à 37°C. Après centrifugation, les *Candida albicans* ont été extraits après addition de triton X 100 et colorés avec une solution de bleu de méthylène à 0,1%. Le pourcentage de *Candida albicans* tués (qui sont uniformément colorés) a été déterminé sous un microscope et les résultats exprimés en pourcentage de lyse.

Produits à l'essai : polysaccharides purs, mis en solution dans le tampon de l'essai (PBS) ; concentrations en µg/ml.

Les résultats exprimés en pourcentage de micro-organismes tués figurent dans le tableau IV ci-après :

**Tableau IV**

| Concentrations | 0 | 0,08 | 0,17 | 0,33 | 0,83 | 1,67 |
|---|---|---|---|---|---|---|
| POL.1 | 2,5 | 4,3 | 7,2 | 10,3 | 12,5 | 12,8 |
| POL.2 | 5 | 6,1 | 9,9 | 13,0 | 16,3 | 16,4 |
| POL.3 | 4,9 | 6,8 | 10,8 | 14,4 | 14,8 | 17,0 |

En conclusion, les trois produits testés présentent donc une activité stimulante intéressante sur la capacité de destruction de ce micro-organisme *Candida albicans* par les kératinocytes humains en culture. Le polysaccharide POL.1 est toutefois moins actif dans ce modèle que les deux autres molécules.

La demanderesse a également conduit des études de cytotoxicité vis-à-vis des principaux germes impliqués dans l'acné (Propionibacterium acnes, Propionibacterium granulosum et staphylococcus epidermis).

En effet, on sait que les peaux jeunes, les peaux à tendance grasse, sont le terrain de prédilection de ces micro-organismes qui prolifèrent de façon anarchique en présence de l'excès de sébum qui caractérise ce type de peaux. C'est un cercle vicieux (excès de sébum, développement de micro-organismes, acné, production accrue de sébum ...) qu'il faut tenter de rompre par le biais du soin cosmétique. Les polysaccharides testés ayant montré une activité stimulante sur la capacité de destruction du *Candida* par les cellules épidermiques, la demanderesse a étudié l'effet de ces polysaccharides sur les 3 germes principaux impliqués dans l'acné. Toutefois rien ne laissait présager qu'une activité sur ces germes là pourrait être mise en évidence.

Une première étude n'a pas montré d'action bactéricide directe des polysaccharides sur les 3 germes en question. Un effet indirect (action des molécules sécrétées par le kératinocyte stimulé) a donc été recherché.

A cet effet, la demanderesse a procédé à une évaluation des effets inducteurs de cytotoxicité des polysaccharides purs vis à vis de *Propionibacterium acnes, Propionibacterium granulosum et Staphylococcus epidermidis* du surnageant de cultures de kératinocytes humains incubés avec différentes doses des produits à l'essai.

Plus précisément, trois doses, respectivement de 0,5µg/ml, 1 µg/ml et 5 µg/ml ont été testées selon un processus comprenant :
- l'incubation des polysaccharides avec des cultures de kératinocytes (3 temps d'incubation respectivement de 15 min, de 1h et de 6 h),
- la récupération du surnageant en le diluant avec du milieu de culture,
- des dilutions successives,
- l'ensemencement des souches de micro-organismes sur géloses, et
- la détermination de la CMI (concentration minimale inhibitrice).

Le temps d'incubation produits à l'essai/kératinocytes qui semble le plus pertinent est le temps 1 heure.

Un effet bactéricide est observé avec les solutions de surnageant de culture les moins diluées. Les trois polysaccharides montrent une activité indirecte sur *Propionibacterium acnes et Staphylococcus epidermidis,* mais ne montrent en revanche aux concentrations testées, aucune activité sur *Propionibacterium granulosum.*

En conclusion, cette étude met en évidence une action destructrice indirecte des polysaccharides à l'essai sur deux des principaux germes impliqués dans le phénomène de l'acné. Là encore, ces polysaccharides se révèlent être d'une aide précieuse pour la peau, lui permettant de réguler, d'équilibrer, sa flore microbienne de surface.

La demanderesse a par ailleurs procédé à une évaluation des effets protecteurs de solutions aqueuses à 1% de polysaccharides (testées aux concentrations de 0,5% à 5%) vis-à-vis des cellules de Langerhans, dans des explants de peau humaine en culture, exposés à une irradiation UVB.

On rappelle que les cellules de Langerhans, cellules dendritiques situées dans l'épiderme, sont des acteurs importants du système de défense immunitaire cutané. Dépourvues de mélanine, ces cellules sont peu protégées des agressions ultraviolettes. Aussi constituent-elles un paramètre très sensible de détection des effets délétères de ces radiations.

Les explants cutanés de 8 mm de diamètre, prélevés à partir d'une plastie abdominale pratiquée chez une femme de 26 ans, ont été mis en culture dans des plaques à 24 puits, contenant 300 µl de milieu par puits, face épidermique vers le haut.

La référence positive consistait en un mélange protecteur composé de vitamine C et de glutathion réduit, en solution aqueuse.

L'application à la surface des épidermes à traiter a été répétée aux temps T24h et T48h.

Au temps T72h, les explants ont été soumis à une irradiation UVB (irradiation totale de 1,5 J/cm²).

Au temps T96h, les explants ont été congelés et des coupes transversales ont été réalisées. Ces coupes-ci traitées par un anticorps antiCD1a (marqueur spécifique des cellules de Langerhans) ont alors été observées au microscope en épifluorescence, et les cellules de Langerhans, fluorescentes, ont été comptées (comptage de 6 champs par lame, soit 12 valeurs par traitement).

Les résultats de cette évaluation sont reportés dans les tableaux V et VI ci-après (le tableau VI indiquant le pourcentage de protection :

**Tableau V**

| | **Produit** | **Explant** | **Nombre de cellules/champs** | | | | | | **Moy** **.** | σ | **%** **viabilité** |
|---|---|---|---|---|---|---|---|---|---|---|---|
| - UVB | Témoin | A | 51 | 58 | 54 | 49 | 53 | 55 | 57 | 4 | 100 |
| | | B | 64 | 60 | 57 | 59 | 58 | 62 | | | |
| + UVB | Témoin | A | 30 | 23 | 17 | 27 | 21 | 24 | 26 | 5 | 46 |
| | | B | 28 | 32 | 21 | 36 | 27 | 24 | | | |
| + UVB | Réf.⊕ | A | 44 | 48 | 44 | 41 | 43 | 47 | 42 | 4 | 73 |
| | | B | 39 | 41 | 34 | 40 | 37 | 40 | | | |
| + UVB | POL.1 | A | 29 | 32 | 37 | 34 | 32 | 31 | 36 | 5 | 63 |
| | 0,5% | B | 46 | 45 | 32 | 36 | 40 | 35 | | | |
| + UVB | POL.1 | A | 49 | 53 | 56 | 56 | 50 | 57 | 50 | 5 | 88 |
| | 5% | B | 52 | 47 | 42 | 49 | 43 | 46 | | | |
| + UVB | POL.2 | A | 44 | 52 | 43 | 39 | 47 | 51 | 45 | 4 | 79 |
| | 0,5% | B | 43 | 46 | 39 | 41 | 46 | 44 | | | |
| + UVB | POL.2 | A | 25 | 24 | 28 | 30 | 25 | 29 | 27 | 2 | 48 |
| | 5% | B | 30 | 26 | 27 | 26 | 24 | 31 | | | |
| + UVB | POL.3 | A | 32 | 37 | 37 | 41 | 39 | 35 | 36 | 3 | 63 |
| | 0,5% | B | 30 | 38 | 36 | 32 | 33 | 37 | | | |
| + UVB | POL.3 | A | 42 | 38 | 47 | 43 | 45 | 40 | 45 | 4 | 80 |
| | 5% | B | 52 | 49 | 44 | 49 | 51 | 42 | | | |

**Tableau VI**

| | **Produit** | **Concentration** | **Nb moyen de cellules** | **%** **Protection** |
|---|---|---|---|---|
| - UVB | Témoin | 0 | 57 | 100 |
| + UVB | Témoin | 0 | 26 | 0 |
| + UVB | Réf.⊕ | 100 µg/ml | 42 | 51 |
| + UVB | Solution POL.1 | 0,5% | 36 | **32** |
| | | 5% | 50 | **78** |
| + UVB | Solution POL.2 | 0,5% | 45 | **61** |
| | | 5% | 27 | 4 |
| + UVB | Solution POL.3 | 0,5% | 36 | **32** |
| | | 5% | 45 | **63** |

En conclusion, cette étude, réalisée sur explants de peau humaine, met en évidence un bon effet protecteur des polysaccharides. Cette protection est particulièrement forte avec les polysaccharides N°1 et N°3. En revanche, on observe avec le polysaccharide N°2, qui montre un effet protecteur excellent à la dose de 0,5% (de solution à 1%), un effet cytotoxique à la dose de 5%. Ce phénomène est sans doute dû à une action cytotoxique conjointe UVB / POL.2 à forte concentration.

Cette démonstration, réalisée dans un modèle expérimental très proche de l'invivo, ne permet pas de conclure sur le mécanisme impliqué dans le phénomène de protection observé. Toutefois, l'ensemble des nombreuses données acquises permet à la demanderesse d'imaginer un mécanisme de protection à la fois direct et indirect sur les cellules de Langerhans :
- Protection antiradicalaire directe (Les cellules de Langerhans étant extrêmement sensibles à l'agression des radicaux libres induits par les rayonnements UV).
- Protection indirecte - Amélioration des mécanismes de défense et de protection des cellules, grâce à la stimulation par les polysaccharides, de la communication cellulaire au sein des explants de peau.

La demanderesse a également procédé à une évaluation de l'effet réducteur sur des animaux (typiquement des cobayes albinos) des polysaccharides POL.1, POL.2, POL.3 en solution aqueuse à 1% de polysaccharides, vis-à-vis de l'érythème induit par les UVB. A cet effet, les animaux ont été exposés à une source émettrice d'ultraviolets B, pendant un temps défini de manière à produire une réaction érythémateuse d'ordre 2. Les produits à l'essai ont été ensuite appliqués sur les zones exposées aux UVB, comparativement à des zones témoins exposées aux UVB et ne recevant pas de produit. Les érythèmes ont été évalués selon l'échelle de cotation suivante :
pas d'érythème = 0
■ taches à peine visibles =0,5
■ érythème léger = 1
■ érythème net = 2
■ érythème très visible = 3

Les résultats de cette évaluation figurent dans le tableau VII ci-après :

**Tableau VII**

| | T + 2h | T + 5h | T + 24h |
|---|---|---|---|
| Témoin | 11 | 13 | 8 |
| Solution POL.1 | 9 | **8** | 8 |
| Solution POL.2 | 12 | **8** | **6** |
| Solution POL.3 | 13 | 11 | 8 |
| | | | |
| Témoin | 10 | 11,5 | 4,5 |
| 5% de solution POL.1 | 7 | **4** | 4,5 |
| 5% de solution POL.2 | 10 | **5,5** | 4,5 |
| 5% de solution POL.3 | 10 | **9,5** | 4 |

En conclusion, cette étude permet de révéler un bon effet apaisant des polysaccharides 1 et 2 suite à une irradiation UVB ("coup de soleil"). En effet ces 2 molécules permettent de réduire considérablement le temps nécessaire à une diminution de l'érythème induit par les UVB. En revanche, le polysaccharide 3 ne se montre pas d 'un grand intérêt dans cette étude.

Des essais d'innocuité ont été en outre conduits avec des solutions aqueuses à 0,1% et 1 % de chacun des trois polysaccharides POL.1, POL.2, POL.3 :
- Essai d'innocuité par administration orale unique (I.V.O.) - Epreuve limite à la dose de 2000 mg/kg, selon J.O. CEE du 24/04/1984 84/449 L251.
- Essai d'irritation primaire cutané (I.P.C.), selon J.O. du 21/02/1982
- Essai de tolérance oculaire (I.O.), selon J.O. du 10/07/1992

Ces essais, dont les résultats figurent dans le tableau VIII ci-après, font apparaître qu'aux concentrations testées, les polysaccharides présentent une innocuité parfaite.

**Tableau VIII**

| | **I.V.O.** | **I.P.C. - classement** | **I.O. - classement** |
|---|---|---|---|
| **POL.1 - 0,1%** | innocuité à la dose de 2000 mg/kg | 0,00 - non irritant | 0,00 - faiblement irritant |
| **POL.1 - 1%** | innocuité à la dose de 2000 mg/kg | 0,00 - non irritant | 0,00 - faiblement irritant |
| **POL.2 - 0,1%** | innocuité à la dose de 2000 mg/kg | 0,00 - non irritant | 0,00 - faiblement irritant |
| **POL.2 - 1%** | innocuité à la dose de 2000 mg/kg | 0,00 - non irritant | 0,00 - faiblement irritant |
| **POL.3 - 0,1%** | innocuité à la dose de 2000 mg/kg | 0,00 - non irritant | 0,00 - faiblement irritant |
| **POL.3 - 1%** | innocuité à la dose de 2000 mg/kg | 0,00 - non irritant | 0,00 - faiblement irritant |

Comme le montrent les différents tests précédemment décrits, les trois polysaccharides testés, issus de la fermentation de bactéries mésophiles d'origine hydrothermale, de nature chimique différente, présentent une activité biologique importante. Cette activité biologique se traduit pour la peau par des effets protecteurs variés :
- protection vis à vis de micro-organismes
- protection des cellules de Langerhans
- action apaisante suite à un "coup de soleil"

De plus l'action stimulante des polysaccharides sur la production de l'Interleukine-1 par les kératinocytes permet d'envisager des vertus cicatrisantes de la part de ces polysaccharides. En effet, l'Interleukine-1 participe plus qu'activement à l'ensemble des processus biologiques impliqués dans la cicatrisation :
- Action chimiotactique directe et indirecte (production de chemokines) : migration des kératinocytes, des monocytes, des lymphocytes...
- Stimulation de la prolifération des kératinocytes, des fibroblastes, des cellules sanguines...
- Stimulation de la synthèse des collagènes et remodelage de la cicatrice.

Tous ces éléments permettent de préconiser l'emploi en cosmétique de ces polysaccharides pour la formulation de produits de soin quotidien pour tout type de peau, visant à préparer la peau, aussi bien que pour la formulation de produits de soin spécifique : produits à visée "anti-âge", produits solaires ou après soleil, produits pour peaux sensibles, produits de soin et de toilette pour les peaux jeunes, les peaux grasses ou les peaux à tendance acnéique, produits pour mains et pieds abîmés, produits après-rasage, produits capillaires traitants, produits de soin du cuir chevelu ...

La concentration en polysaccharide préconisée pour un emploi cosmétique, se situera entre 0,001% et 5%, en fonction de l'activité recherchée et du type de formulation mise en oeuvre.

Les polysaccharides utilisés pourront être natifs (c'est à dire non modifiés physiquement ou chimiquement). Ils pourront être dépolymérisés, en des fractions de poids moléculaire faible ou important. Ils pourront également être modifiés chimiquement (sulfatés, acidifiés, azotés). Ils pourront se présenter sous forme lyophilisée, sous forme de solutions plus ou moins gélifiées, ou encore sous forme encapsulée.

Bien entendu, les compositions selon l'invention pourront se présenter sous la forme d'émulsions simples ou multiples (crèmes ou laits eau/huile ou huile/eau, émulsions triples, micro-émulsions, émulsions à cristaux liquides), de gels aqueux ou huileux, de lotions aqueuses, huileuses, hydroalcooliques ou biphasiques, de sticks, ou de poudres ou de tout système vectorisé (systèmes « à libération contrôlée » ou systèmes à « libération modulée »). Elles seront utilisées par voie topique.

Des exemples de formulations de la composition cosmétique seront décrits, ci-après, à titre d'exemple non limitatif:

### Gel hydratant peaux à tendance acnéique

■ Aqua QSP 100%
■ Alcool 96° 5 à 10%
■ Cyclomethicone 2 à 10%
■ Glycérine 1 à 5%
■ Sclerotium gum 0,3 à 0,6%
■ Carbomer 0,2 à 0,5%
■ Triethanolamine 0,2 à 0,5%
■ Conservateur antimicrobien 0,1 à 0,7%
■ Parfum 0,1 à 0,5%
■ PPG-26 buteth-26 & PEG-40 hydrogenated castor oil 0,1 à 0,5 %
■ Polysaccharide issu d'une fermentation de bactéries mésophiles hydrothermales 0,001 à 5%

### Crème solaire

■ Aqua QSP 100%
■ Octylmethoxycinnamate 5 à 10%
■ Isopropyl lanolate 2 à 5%
■ Myreth-3myristate 2 à 5%
■ PEG-6 stearate & ceteth-20 & glyceryl stearate & steareth-20 2 à 5%
■ Butyl methoxy dibenzoyl methane 1 à 5%
■ huile végétale 1 à 5%
■ Nylon-12 1 à 5%
■ Glycérine 1 à 5%
■ Stearyl dimethicone 0,5 à 3%
■ Carbomer 0,2 à 0,6%
■ Triéthanolamine 0,2 à 0,6%
■ Conservateur antimicrobien 0,1 à 0,7%
■ Parfum 0,1 à 0,5%
■ Tétrasodium EDTA 0,05 à 0,15%
■ BHT 0,01 à 0,05%
■ Polysaccharide issu d'une fermentation de bactéries mésophiles hydrothermales 0,001 à 5%

### Lait après soleil

■ Aqua QSP 100%
■ Dimethicone copolyol 2 à 5%
■ Propylène glycol 2 à 5%
■ Myreth-3 myristate 2 à 5%
■ Huile végétale 2 à 5%
■ Huile minérale 2 à 5%
■ Dimethicone 2 à 5%
■ Polysorbate 60 2 à 3%
■ Sorbitan stearate 2 à 3%
■ Isopropyle lanolate 1 à 4%
■ Aluminum starch octenyl-succinate 1 à 3%
■ Acrylates / C10-30 alkyl acrylate crosspolymer 0,2 à 0,5%
■ Triéthanolamine 0,2 à 0,5%
■ Conservateur antimicrobien 0,1 à 0,7%
■ Parfum 0,1 à 0,5%
■ Tétrasodium EDTA 0,05 à 0,15%
■ BHT 0,01 à 0,05%
■ Polysaccharide issu d'une fermentation de bactéries mésophiles hydrothermales 0,001 à 5%

### Sérum anti-âge

■ Aqua QSP 100%
■ Huile minérale 5 à 10%
■ Dimethicone 1 à 3%
■ Acrylates / C10-30 alkyl acrylatecrosspolymer 0,2 à 0,5%
■ Triethanolamine 0,2 à 0,5%
■ Conservateur antimicrobien 0,1 à 0,7%
■ Parfum 0,1 à 0,5%
■ Polysaccharide issu d'une fermentation de bactéries mésophiles hydrothermales 0,001 à 5%

### Shampooing régulateur

■ Aqua QSP 100%
■ Sodium Laureth sulfate 10 à 20%
■ Cocamidopropyl betaine 5 à 15%
■ Caprylyl / capryl glucoside 5 à 10%
■ Cocamide DEA 2 à 5%
■ Acrylate / steareth-20 metacrylate copolymer 1 à 4%
■ Glycérine 1 à 3%
■ PEG-120 methyl glucose dioleate 0,5 à 2%
■ Parfum 0,2 à 1%
■ Conservateur antimicrobien 0,1 à 0,7%
■ Tétrasodium EDTA 0,05 à 0,15%
■ Polysaccharide issu d'une fermentation de bactéries mésophiles hydrothermales 0,001 à 5%

## Revendications

1. Composition cosmétique,
**caractérisée en ce qu'**elle comprend au moins un polysaccharide provenant de bactéries d'origine hydrothermale, ce polysaccharide de poids moléculaire élevé de 100 000 à 1 million de daltons étant présent dans la composition en une concentration comprise entre 0,001 % et 5 %.

2. Composition selon la revendication 1,
**caractérisée en ce que** le susdit polysaccharide est issu de la fermentation desdites bactéries.

3. Composition selon la revendication 1,
**caractérisée en ce que** le susdit polysaccharide est un exopolysaccharide.

4. Composition selon l'une des revendications précédentes,
**caractérisée en ce que** les susdites bactéries sont des bactéries mésophiles.

5. Composition selon l'une des revendications précédentes,
**caractérisée en ce que** les polysaccharides sont natifs.

6. Composition selon l'une des revendications 1 à 4,
**caractérisée en ce que** les polysaccharides sont dépolymérisées en des fractions de poids moléculaire faible ou important.

7. Composition selon l'une des revendications 1 à 4,
**caractérisée en ce que** les polysaccharides sont modifiés chimiquement.

8. Composition selon la revendication 7,
**caractérisée en ce que** le polysaccharide est sulfaté, acidifié ou azoté.

9. Composition selon l'une des revendications précédentes,
**caractérisée en ce que** le polysaccharide se présente sous forme lyophilisée, sous forme de solution, ou encore sous forme encapsulée.

10. Composition selon l'une des revendications précédentes,
**caractérisée en ce qu'**elle est utilisée par voie topique et se présente sous la forme d'émulsions simples ou multiples, de gels aqueux ou huileux, de lotions aqueuses, huileuses, hydroalcooliques ou biphasiques, de sticks, de poudres ou de système vectorisé.

11. Composition selon l'une des revendications précédentes,
**caractérisée en ce qu'**elle s'applique aux produits de soin quotidien pour tout type de peau.

12. Composition selon l'une des revendications 1 à 10,
**caractérisée en ce qu'**elle s'applique aux soins anti-âge.

13. Composition selon l'une des revendications 1 à 10,
**caractérisée en ce qu'**elle s'applique aux produits solaires, ou après-soleil.

14. Composition selon l'une des revendications 1 à 10,
**caractérisée en ce qu'**elle s'applique aux produits pour peaux sensibles.

15. Composition selon l'une des revendications 1 à 10,
**caractérisée en ce qu'**elle s'applique aux produits de soins et de toilette pour les peaux jeunes, les peaux grasses ou les peaux à tendance acnéique.

16. Composition selon l'une des revendications 1 à 10,
**caractérisée en ce qu'**elle s'applique aux produits pour mains et pieds abîmés.

17. Composition selon l'une des revendications 1 à 10,
**caractérisée en ce qu'**elle s'applique aux produits après-rasage.

18. Composition selon l'une des revendications 1 à 10,
**caractérisée en ce qu'**elle s'applique aux produits capillaires traitants.

19. Composition selon l'une des revendications 1 à 10,
**caractérisée en ce qu'**elle s'applique aux produits de soi du cuir chevelu.

20. Gel hydratant pour peaux à tendance acnéique,
**caractérisé en ce qu'**il présente au moins la composition suivante :
■ Aqua QSP 100%
■ Alcool 96° 5 à 10%
■ Cyclomethicone 2 à 10%
■ Glycérine 1 à 5%
■ Sclerotium gum 0,3 à 0,6%
■ Carbomer 0,2 à 0,5%
■ Triethanolamine 0,2 à 0,5%
■ Conservateur antimicrobien 0,1 à 0,7%
■ Parfum 0,1 à 0,5%
■ PPG-26 buteth-26 & PEG-40 hydrogenated castor oil 0,1 à 0,5 %
■ Polysaccharide issu d'une fermentation de bactéries mésophiles hydrothermales 0,001 à 5%

21. Crème solaire,
**caractérisée en ce qu'**elle présente la composition suivante :
Aqua QSP 100%
■ Octyl methoxycinnamate 5 à 10%
■ Isopropyl lanolate 2 à 5%
■ Myreth-3 myristate 2 à 5%
■ PEG-6 stearate & ceteth-20 & glyceryl stearate & steareth-20 2 à 5%
■ Butyl methoxy dibenzoyl méthane 1 à 5%
■ Huile végétale 1 à 5%
■ Nylon-12 1 à 5%
■ glycérine 1 à 5%
■ Stearyl dimethicone 0,5 à 3%
■ Carbomer 0,2 à 0,6%
■ Triéthanolamine 0,2 à 0,6%
■ Conservateur antimicrobien 0,1 à 0,7%
■ Parfum 0,1 à 0,5%
■ Tétrasodium EDTA 0,05 à 0,15%
■ BHT 0,01 à 0,05%
■ Polysaccharide issu d'une fermentation de bactéries mésophiles hydrothermales 0,001 à 5%

22. Lait après soleil,
**caractérisé en ce qu'**il présente la composition suivante :
■ Aqua QSP 100%
■ Dimethicone copolyol 2 à 5%
■ Propylène glycol 2 à 5%
■ Myreth-3 myristate 2 à 5%
■ Huile végétale 2 à 5%
■ Huile minérale 2 à 5%
■ Dimethicone 2 à 5%
■ Polysorbate 60 2 à 3%
■ Sorbitan stearate 2 à 3%
■ Isopropyle lanolate 1 à 4%
■ Aluminum starch octenyl-succinate 1 à 3%
■ Acrylates / C10-30 alkyl acrylate crosspolymer 0,2 à 0,5%
■ Triéthanolamine 0,2 à 0,5%
■ Conservateur antimicrobien 0,1 à 0,7%
■ Parfum 0,1 à 0,5%
■ Tétrasodium EDTA 0,05 à 0,15%
■ BHT 0,01 à 0,05%
■ Polysaccharide issu d'une fermentation de bactéries mésophiles hydrothermales 0,001 à 5%

23. Sérum anti-âge,
**caractérisé en ce qu'**il présente la composition suivante :
■ Aqua QSP 100%
■ Huile minérale 5 à 10%
■ Dimethicone 1 à 3%
■ Acrylates / C10-30 alkyl acrylatecrosspolymer 0,2 à 0,5%
■ Triethanolamine 0,2 à 0,5%
■ Conservateur antimicrobien 0,1 à 0,7%
■ Parfum 0,1 à 0,5%
■ Polysaccharide issu d'une fermentation de bactéries mésophiles hydrothermales 0,001 à 5%

24. Shampooing régulateur,
**caractérisé en ce qu'**il présente la composition suivante :
■ Aqua QSP 100%
■ Sodium Laureth sulfate 10 à 20%
■ Cocamidopropyl betaine 5 à 15%
■ Caprylyl / capryl glucoside 5 à 10%
■ Cocamide DEA 2 à 5%
■ Acrylate / steareth-20 metacrylate copolymer 1 à 4%
■ Glycérine 1 à 3%
■ PEG-120 methyl glucose dioleate 0,5 à 2%
■ Parfum 0,2 à 1%
■ Conservateur antimicrobien 0,1 à 0,7%
■ Tétrasodium EDTA 0,05 à 0,15%
■ Polysaccharide issu d'une fermentation de bactéries mésophiles hydrothermales 0,001 à 5%

## Patentansprüche

1. Kosmetikzusammensetzung, **dadurch gekennzeichnet, daß** sie mindestens ein Polysaccharid, das von Bakterien hydrothermalen Ursprungs stammt, enthält, wobei dieses hochmolekulare Polysaccharid von 100 000 bis 1 000 000 Dalton in der Zusammensetzung in einer Konzentration zwischen 0,001% und 5% vorliegt.

2. Zusammensetzung nach Anspruch 1, **dadurch gekennzeichnet, daß** das obengenannte Polysaccharid durch Fermentation dieser Bakterien gewonnen wird.

3. Zusammensetzung nach Anspruch 1, **dadurch gekennzeichnet, daß** es sich bei dem obengenannten Polysaccharid um ein Exopolysaccharid handelt.

4. Zusammensetzung nach einem der vorhergehenden Ansprüche, **dadurch gekennzeichnet, daß** es sich bei den obengenannten Bakterien um mesophile Bakterien handelt.

5. Zusammensetzung nach einem der vorhergehenden Ansprüche, **dadurch gekennzeichnet, daß** die Polysaccharide nativ sind.

6. Zusammensetzung nach einem der Ansprüche 1 bis 4, **dadurch gekennzeichnet, daß** die Polysaccharide in hoch- oder niedermolekulare Fraktionen entpolymerisiert sind.

7. Zusammensetzung nach einem der Ansprüche 1 bis 4, **dadurch gekennzeichnet, daß** die Polysaccharide chemisch modifiziert sind.

8. Zusammensetzung nach Anspruch 7, **dadurch gekennzeichnet, daß** das Polysaccharid sulfathaltig, säurehaltig oder stickstoffhaltig ist.

9. Zusammensetzung nach einem der vorhergehenden Ansprüche, **dadurch gekennzeichnet, daß** das Polysaccharid in lyophilisierter Form, in Form einer Lösung oder in eingekapselter Form vorliegt.

10. Zusammensetzung nach einem der vorhergehenden Ansprüche, **dadurch gekennzeichnet, daß** sie topisch angewendet wird und in Form von einfachen oder multiplen Emulsionen, wäßrigen oder öligen Gels, wäßrigen, öligen, wäßrig-alkoholischen oder zweiphasigen Lotionen, Stiften, Pudern oder einem Trägersystem vorliegt.

11. Zusammensetzung nach einem der vorhergehenden Ansprüche, **dadurch gekennzeichnet, daß** sie bei Produkten zur täglichen Pflege für jeden Hauttyp angewandt wird.

12. Zusammensetzung nach einem der Ansprüche 1 bis 10, **dadurch gekennzeichnet, daß** sie bei Mitteln gegen Alterungserscheinungen angewandt wird.

13. Zusammensetzung nach einem der Ansprüche 1 bis 10, **dadurch gekennzeichnet, daß** sie bei Sonnenschutzmitteln oder After-Sun-Mitteln angewandt wird.

14. Zusammensetzung nach einem der Ansprüche 1 bis 10, **dadurch gekennzeichnet, daß** sie bei Produkten für empfindliche Haut angewandt wird.

15. Zusammensetzung nach einem der Ansprüche 1 bis 10, **dadurch gekennzeichnet, daß** sie bei Pflege- und Waschprodukten für jugendliche Haut, fettige Haut oder Haut, die zu Akne neigt, angewandt wird.

16. Zusammensetzung nach einem der Ansprüche 1 bis 10, **dadurch gekennzeichnet, daß** sie bei Produkten für geschädigte Hände und Füße angewandt wird.

17. Zusammensetzung nach einem der Ansprüche 1 bis 10, **dadurch gekennzeichnet, daß** sie bei After-Shave-Produkten angewandt wird.

18. Zusammensetzung nach einem der Ansprüche 1 bis 10, **dadurch gekennzeichnet, daß** sie bei Haarpflegeprodukten angewandt wird.

19. Zusammensetzung nach einem der Ansprüche 1 bis 10, **dadurch gekennzeichnet, daß** sie bei Produkten zur Pflege der Kopfhaut angewandt wird.

20. Feuchtigkeitspendendes Gel für Haut, die zu Akne neigt, **dadurch gekennzeichnet, daß** es mindestens die folgende Zusammensetzung aufweist:
• Wasser auf 100%
• Alkohol 96%ig 5 bis 10%
• Cyclometikon 2 bis 10%
• Glycerin 1 bis 5%
• Sclerotium Gum 0,3 bis 0,6%
• Carbomer 0,2 bis 0,5%
• Triethanolamin 0,2 bis 0,5%
• Konservierungsstoff Mikroorganismen gegen 0,1 bis 0,7%
• Duftstoff 0,1 bis 0,5%
• PPG-26 Buteth-26 & PEG-40 0,1 bis 0,5% hydriertes Rizinusöl
• Polysaccharid, das durch Fermentation von mesophilen hydrothermalen Bakterien gewonnen wird 0,001 bis 5%

21. Sonnencreme, **dadurch gekennzeichnet, daß** sie die folgende Zusammensetzung aufweist:
• Wasser auf 100%
• Octylmethoxycinnamat 5 bis 10%
• Isopropyllanolat 2 bis 5%
• Myreth-3-myristat 2 bis 5%
• PEG-6 Stearat & Ceteth-20 & Glycerylstearat & Steareth-20 2 bis 5%
• Butylmethoxydibenzoylmethan 1 bis 5%
• Pflanzenöl 1 bis 5%
• Nylon-12 1 bis 5%
• Glycerin 1 bis 5%
• Stearyldimetikon 0,5 bis 3%
• Carbomer 0,2 bis 0,6%
• Triethanolamin 0,2 bis 0,6%
• Konservierungsmittel gegen Mikroorganismen 0,1 bis 0,7%
• Duftstoff 0,1 bis 0,5%
• EDTA-Tetranatriumsalz 0,05 bis 0,15%
• BHT 0,01 bis 0,05%
• Polysaccharid, das durch Fermentation von mesophilen hydrothermalen Bakterien gewonnen wird 0,001 bis 5%

22. After-Sun-Milch, **dadurch gekennzeichnet, daß** sie die folgende Zusammensetzung aufweist:
• Wasser auf 100%
• Dimetikoncopolyol 2 bis 5%
• Propylenglykol 2 bis 5%
• Myreth-3-myristat 2 bis 5%
• Pflanzenöl 2 bis 5%
• Mineralöl 2 bis 5%
• Dimetikon 2 bis 5%
• Polysorbat 60 2 bis 3%
• Sorbitanstearat 2 bis 3%
• Isopropyllanolat 1 bis 4%
• Aluminiumstärkeoctenyl-succinat 1 bis 3%
• Acrylate/C₁₀₋₃₀-Alkylacrylat- 0,2 bis 0,5%
• Triethanolamin 0,2 bis 0,5%
• Konservierungsmittel gegen Mikroorganismen 0,1 bis 0,7%
• Duftstoff 0,1 bis 0,5%
• EDTA-Tetranatriumsalz 0,05 bis 0,15%
• BHT 0,01 bis 0,05%
• Polysaccharid, das durch Fermentation von mesophilen hydrothermalen Bakterien gewonnen wird 0,001 bis 5%

23. Serum gegen Alterungserscheinungen, **dadurch gekennzeichnet, daß** es die folgende Zusammensetzung aufweist:
• Wasser auf 100%
• Mineralöl 5 bis 10%
• Dimetikon 1 bis 3%
• Acrylate/C₁₀₋₃₀-Alkylacrylat-Crosspolymer 0,2 bis 0,5%
• Triethanolamin 0,2 bis 0,5%
• Konservierungsmittel gegen 0,1 bis 0,7% Mikroorganismen
• Duftstoff 0,1 bis 0,5%
• Polysaccharid, das durch Fermentation von mesophilen hydrothermalen Bakterien gewonnen wird 0,001 bis 5%

24. Regulierendes Shampoo, **dadurch gekennzeichnet, daß** es die folgende Zusammensetzung aufweist:
• Wasser auf 100%
• Natriumlaurethsulfat 10 bis 20%
• Cocamidopropylbetain 5 bis 15%
• Caprylyl/Caprylglucosid 5 bis 10%
• Cocamid DEA 2 bis 5%
• Acrylat/Steareth-20 Methacrylat-Copolymer 1 bis 4%
• Glycerin 1 bis 3%
• PEG-120-Methylglucose-dioleat 0,5 bis 2%
• Duftstoff 0,2 bis 1%
• Konservierungsmittel gegen 0,1 bis 0,7% Mikroorganismen
• EDTA-Tetranatriumsalz 0,05 bis 0,15%
• Polysaccharid, das durch Fermentation von mesophilen hydrothermalen Bakterien gewonnen wird 0,001 bis 5%

## Claims

1. A cosmetic composition,
**characterized in that** it comprises at least one polysaccharide derived from bacteria of hydrothermal origin, this polysaccharide high molecular weight of from 100,000 to 1 million Daltons, being present in the composition in concentration comprised between 0.001% and 5%.

2. A composition according to claim 1,
**characterized in that** the abovementioned polysaccharide is derived from fermenting the said bacteria.

3. A composition according to claim 1,
**characterized in that** the abovementioned polysaccharide is an exopolysaccharide.

4. A composition according to one of the preceding claims,
**characterized in that** the abovementioned bacteria are mesophilic bacteria.

5. A composition according to one of the preceding claims,
**characterized in that** the polysaccharides are native polysaccharides.

6. A composition according to one of the claims 1 to 4,
**characterized in that** the polysaccharides are depolymerized into fractions of low molecular weight or of substantial molecular weight.

7. A composition according to one of the claims 1 to 4,
**characterized in that** the polysaccharides are modified chemically.

8. A composition according to claim 7,
**characterized in that** the polysaccharide is sulfated, acidified or nitrogenized.

9. A composition according to one of the preceding claims,
**characterized in that** the polysaccharide is present in lyophilized form, in solution form, or yet again in encapsulated form.

10. A composition according to one of the preceding claims,
**characterized in that** it is used topically and which is present in the form of simple or complex emulsions, of aqueous or oily gels, of aqueous, oily, hydroalcoholic or biphasic lotions, of sticks, of powders or of a vectorized system.

11. A composition according to one of the preceding claims,
**characterized in that** it applies to everyday care products for all types of skin.

12. A composition according to one of the claims 1 to 10,
**characterized in that** it applies to anti-aging care.

13. A composition according to one of the claims 1 to 10,
**characterized in that** it applies to sunscreen or after-sun products.

14. A composition according to one of the claims 1 to 10,
**characterized in that** it applies to products for sensitive skins.

15. A composition according to one of the claims 1 to 10,
**characterized in that** it applies to care and cleansing products for young skins, greasy skins or skins having a tendency to acne.

16. A composition according to one of the claims 1 to 10,
**characterized in that** it applies to products for damaged hands and feet.

17. A composition according to one of the claims 1 to 10,
**characterized in that** applies to aftershave products.

18. A composition according to one of the claims 1 to 10,
**characterized in that** it applies to hair-treatment products.

19. A composition according to one of the claims 1 to 10,
**characterized in that** it applies to scalp care products.

20. A moisturizing gel for skins having a tendency to acne,
**characterized in that** it possesses at least the following composition:
• Water QS for 100%
• 96° alcohol 5 to 10%
• Cyclomethicone 2 to 10%
• Glycerol 1 to 5%
• Sclerotium gum 0.3 to 0.6%
• Carbomer 0.2 to 0.5%
• Triethanolamine 0.2 to 0.5%
• Antimicrobial preservative 0.1 to 0.7%
• Perfume 0.1 to 0.5%
• PPG-26 buteth-26 & PEG-40 hydrogenated castor oil 0.1 to 0.5%
• Polysaccharide derived from a fermentation of mesophilic hydrothermal bacteria 0.001 to 5%

21. A suncream,
**characterized in that** it possesses the following composition:
• Water QS for 100%
• Octyl methoxycinnamate 5 to 10%
• Isopropyl lanolate 2 to 5%
• Myreth-3 myristate 2 to 5%
• PEG-6 stearate & ceteth-20 & glyceryl stearate & steareth-20 2 to 5%
• Butylmethoxydibenzoylmethane 1 to 5%
• Vegetable oil 1 to 5%
• Nylon-12 1 to 5%
• Glycerol 1 to 5%
• Stearyl dimethicone 0.5 to 3%
• Carbomer 0.2 to 0.6%
• Triethanolamine 0.2 to 0.6%
• Antimicrobial preservative 0.1 to 0.7%
• Perfume 0.1 to 0.5%
• Tetrasodium EDTA 0.05 to 0.15%
• BHT 0.01 to 0.05%
• Polysaccharide derived from a fermentation of mesophilic hydrothermal bacteria 0.001 to 5%

22. An after-sun lotion,
**characterized in that** it possesses the following composition:
• Water QS for 100%
• Dimethicone copolyol 2 to 5%
• Propylene glycol 2 to 5%
• Myreth-3 myristate 2 to 5%
• Vegetable oil 2 to 5%
• Mineral oil 2 to 5%
• Dimethicone 2 to 5%
• Polysorbate 60 2 to 3%
• Sorbitan stearate 2 to 3%
• Isopropyl lanolate 1 to 4%
• Aluminum starch octenyl-succinate 1 to 3%
• Acrylates/C10-30 alkyl acrylate crosspolymer 0.2 to 0.5%
• Triethanolamine 0.2 to 0.5%
• Antimicrobial preservative 0.1 to 0.7%
• Perfume 0.1 to 0.5%
• Tetrasodium EDTA 0.05 to 0.15%
• BHT 0.01 to 0.05%
• Polysaccharide derived from a fermentation of mesophilic hydrothermal bacteria 0.001 to 5%

23. An anti-aging serum,
**characterized in that** it possesses the following composition:
• Water QS for 100%
• Mineral oil 5 to 10%
• Dimethicone 1 to 3%
• Acrylates/C10-30 alkyl acrylate crosspolymer 0. 2 to 0.5%
• Triethanolamine 0.2 to 0.5%
• Antimicrobial preservative 0.1 to 0.7%
• Perfume 0.1 to 0.5%
• Polysaccharide derived from a fermentation of mesophilic hydrothermal bacteria 0.001 to 5%

24. A regulating shampoo,
**characterized in that** it possesses the following composition:
• Water QS for 100%
• Sodium laureth sulfate 10 to 20%
• Cocamidopropyl betaine 5 to 15%
• Caprylyl/capryl glucoside 5 to 10%
• Cocamide DEA 2 to 5%
• Acrylate/steareth-20 methacrylate copolymer 1 to 4%
• Glycerol 1 to 3%
• PEG-120 methyl glucose dioleate 0.5 to 2%
• Perfume 0.2 to 1%
• Antimicrobial preservative 0.1 to 0.7%
• Tetrasodium EDTA 0.05 to 0.15%
• Polysaccharide derived from a fermentation of mesophilic hydrothermal bacteria 0.001 to 5%
